# EUROPEAN PATENT APPLICATION

(11) **EP 4 574 162 A2**
(43) Date of publication of application: **25.06.2025**
(21) Application number: 25169364.4
(22) Date of filing: 12.08.2021
(51) Int. Cl.: A61K 38/04

(54) **BONE MORPHOGENETIC PROTEIN-9 AND -10 VARIANTS WITH ENHANCED THERAPEUTIC EFFECT DUE TO REDUCED SIDE EFFECTS OF ECTOPIC OSSIFICATION AND PHARMACEUTICAL COMPOSITION COMPRISING SAME**

(30) Priority: 13.08.2020 KR 20200101759
(62) Divisional of application: 21856266.8
(71) Applicant: Nibec Co., Ltd., Chungcheongbuk-do 27816 (KR)
(72) Inventor: CHUNG, Chong-Pyoung, 05618 Seoul (KR); PARK, Yoon Jeong, 08291 Seoul (KR); LEE, Jue-Yeon, 13831 Gwacheon-si Gyeonggi-do (KR); YOON, Gook-Jin, 05659 Seoul (KR); LEE, Dong Woo, 08652 Seoul (KR)
(74) Representative: Guardian IP Consulting I/S

(57) **Abstract**

The present invention relates to a BMP-9 variant and a derivative thereof. The variant stimulates endothelial cell-specific signaling, but does not stimulate ectopic ossification-related signaling, as compared to wild-type BMP-9, and thus has the effects of enhancing therapeutic effects on various diseases, including tumors, cardiovascular disease, fibrotic diseases, inflammatory diseases, metabolic diseases, and autoimmune diseases, and reducing side effects.

## Description

### Technical Field

The present invention relates to a BMP-9 variant and a derivative thereof, and more particularly to a BMP-9 variant and a derivative thereof, in which side effects of ectopic ossification are reduced and therapeutic effects are enhanced in the treatment of various diseases, disorders and conditions, including tumors, cardiovascular diseases, fibrotic diseases, and metabolic diseases.

### Background Art

BMP-9 binds to a unique receptor, rather than binding to conventionally known BMP receptors, and is distinguished from other types of BMPs because of diverse roles thereof in a variety of intracellular processes. For example, BMP-9 is produced in the liver and is able to suppress lipid metabolism, control hepatic glucose production, regulate the growth and migration of endothelial cells in the cardiovascular system, and inhibit cardiomyoblast fibrosis. In particular, in the cardiovascular system, together with BMP-10, BMP-9 binds to endothelial ALK-1 receptors, BMPR-II, and endoglin, and is thus involved in vascular homeostasis and blood pressure regulation. In many documents, it has been reported that, when BMP-9 and BMP-10 are applied to pulmonary arterial hypertension or myocardial fibrosis caused by a deletion of the corresponding receptor, the symptoms are alleviated, indicating high utility of BMP-9 as a therapeutic material. Moreover, BMP-9 is one of powerful BMPs that may induce the death of prostate cancer cells depending on the type of tumor and may regulate osteogenic differentiation in bone tissue. In addition, BMP-9 is known to have high potential as a new anti-diabetic or anti-obesity therapeutic target because it is effective at improving insulin sensitivity.

Accordingly, for the development of new therapeutic agents targeting growth factors including BMP-9, attempts have been made to increase the biological activity of growth factors by substituting, introducing, and removing some amino acids of wild-type growth factors to create mutations (WO 2010/065439). However, a problem associated with the direct use of growth factors including BMP-9 as biotherapeutic agents is that ectopic ossification and deformity growth factor action are simultaneously expressed. This is due to the complexity of receptors of growth factors including BMP-9, and there are still few cases of alleviating side effects related to ectopic ossification. The receptors of BMP-9 and BMP-10 are mainly present in vascular endothelial cells, and are known to be involved in vascular function regulation while acting as ligands of Alk-1 and BMPR-II. Specifically, BMPR-II forms a complex with ALK-1 and selectively reacts with BMP-9 or BMP-10. As such, in the case in which the receptor is deleted or BMP-9 or BMP-10 is deficient, the most frequent diseases are pulmonary arterial hypertension and myocardial fibrosis. In particular, BMP-9 acts directly on endothelial cells to enhance the integrity of the inner wall of blood vessels, thus increasing vascular stability, thereby inhibiting vascular cell death or angiogenesis. Moreover, unlike other types of BMPs, such as BMP-2, BMP-4, BMP-6, etc., BMP-9 is known to promote endothelial cell activity even at low concentrations that do not cause ossification. However, BMP-9 may also induce ectopic ossification at high concentrations, and as such, the application of variants in which the ectopic ossification function is controlled is required in actual clinical practice.

In addition, it has been pointed out that the very short *in-vivo* half-life of the wild type due to the properties of growth factors makes commercialization as a therapeutic agent difficult (Kharitonenkov A. et al., Journal of Clinical Investigation, 115:1627-1635, 2005). BMP-9 has a short *in-vivo* half-life of 10 minutes to 1 hour in mice and 1.5 hours to 2 hours in monkeys, and if it is developed as a therapeutic agent, it has the disadvantage of having to administer the same daily. To date, various techniques have been reported to increase the *in-vivo* half-life of recombinant proteins. There is an example in which a protein is linked with polyethylene glycol (PEG), which is a polymer material, so that the molecular weight thereof is increased, thereby inhibiting renal excretion, ultimately increasing the residence time *in vivo* (WO 2012/066075). Also, there is an example in which the half-life is increased by fusing a fatty acid that binds to human albumin to a growth factor molecule (WO 2012/010553). Furthermore, there is an example of increasing the half-life while exhibiting the same pharmacological activity as the mechanism of action of the growth factor by synthesizing an agonistic antibody that specifically binds to a human growth factor receptor alone or a complex with beta-Klotho (WO 2012/170438). In addition, there is an example in which the half-life is increased by synthesizing a long-acting fusion protein in which Fc of immunoglobulin IgG is linked to a growth factor molecule (WO 2013/188181).

Accordingly, the present inventors have made great efforts on the study of BMP9-based therapeutic variant proteins to inhibit ectopic ossification and extend the action time, and thus ascertained that some variants may inhibit ectopic ossification and have increased *in-vivo* half-life, thereby culminating in the present invention.

The information described in this background section is only for improving understanding of the background of the present invention, and is not to be construed as including information forming the related art already known to those of ordinary skill in the art to which the present invention belongs.

### Prior art

### Patent Literature

(Patent Document 1) WO 2010/065439
(Patent Document 2) WO 2012/066075
(Patent Document 3) WO 2012/010553
(Patent Document 4) WO 2012/170438
(Patent Document 5) WO 2013/188181

### Non-Patent Literature

(Non-Patent Document 1) Kharitonenkov A. et al., Journal of Clinical Investigation, 115:1627-1635, 2005

### Summary of the Invention

It is an object of the present invention to provide a bone morphogenetic protein-9 (BMP-9) variant and a fusion thereof with reduced side effects of ectopic ossification.

It is another object of the present invention to provide various therapeutic uses of the bone morphogenetic protein-9 (BMP-9) variant and the fusion thereof.

It is still another object of the present invention to provide a pharmaceutical composition for preventing or treating tumors, inflammatory diseases, metabolic diseases, or autoimmune diseases, comprising the BMP-9 variant or the fusion thereof.

It is yet another object of the present invention to provide a method of preventing or treating tumors, inflammatory diseases, metabolic diseases, or autoimmune diseases, comprising administering the BMP-9 variant or the fusion thereof.

It is still yet another object of the present invention to provide the use of the BMP-9 variant and the fusion thereof for the prevention or treatment of tumors, inflammatory diseases, metabolic diseases, or autoimmune diseases.

It is even yet another object of the present invention to provide the use of the BMP-9 variant and the fusion thereof for the manufacture of a medicament for the treatment of tumors, inflammatory diseases, metabolic diseases, or autoimmune diseases.

In order to accomplish the above objects, the present invention provides a bone morphogenetic protein-9 (BMP-9) variant represented by any one amino acid sequence selected from the group consisting of SEQ ID NOs: 2 to 29.

In addition, the present invention provides a BMP-9 variant-Fc fusion protein in which an Fc fragment of an immunoglobulin is linked to the BMP-9 variant.

In addition, the present invention provides a pharmaceutical composition for treating a tumor comprising the BMP-9 variant or the BMP-9 variant-Fc fusion protein as an active ingredient.

In addition, the present invention provides a pharmaceutical composition for treating an inflammatory disease comprising the BMP-9 variant or the BMP-9 variant-Fc fusion protein as an active ingredient.

In addition, the present invention provides a pharmaceutical composition for treating a metabolic disease comprising the BMP-9 variant or the BMP-9 variant-Fc fusion protein as an active ingredient.

In addition, the present invention provides a pharmaceutical composition for treating an autoimmune disease comprising the BMP-9 variant or the BMP-9 variant-Fc fusion protein as an active ingredient.

In addition, the present invention provides a method of preventing or treating a tumor, an inflammatory disease, a metabolic disease, or an autoimmune disease, comprising administering the BMP-9 variant or the BMP-9 variant-Fc fusion protein.

In addition, the present invention provides the use of the BMP-9 variant or the BMP-9 variant-Fc fusion protein for the prevention or treatment of a tumor, an inflammatory disease, a metabolic disease, or an autoimmune disease.

In addition, the present invention provides the use of the BMP-9 variant or the BMP-9 variant-Fc fusion protein for the manufacture of a medicament for the treatment of a tumor, an inflammatory disease, a metabolic disease, or an autoimmune disease.

### Brief Description of Drawings

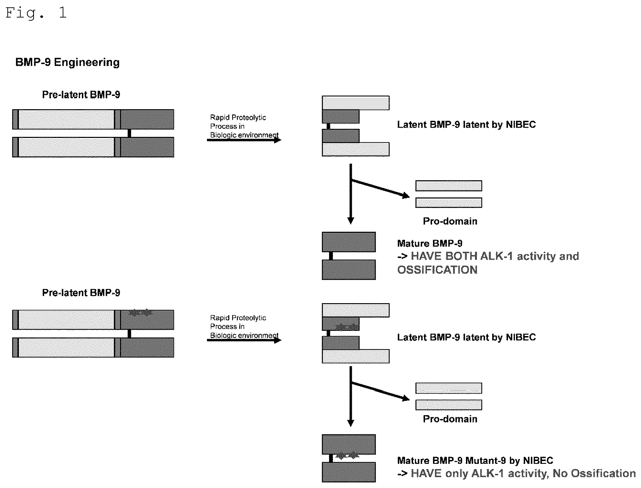

FIG. 1 schematically shows the outline of the present invention.
FIG. 2 shows an expression vector into which BMP-9, a BMP-9 variant, and Fc-fused BMP-9 are introduced.

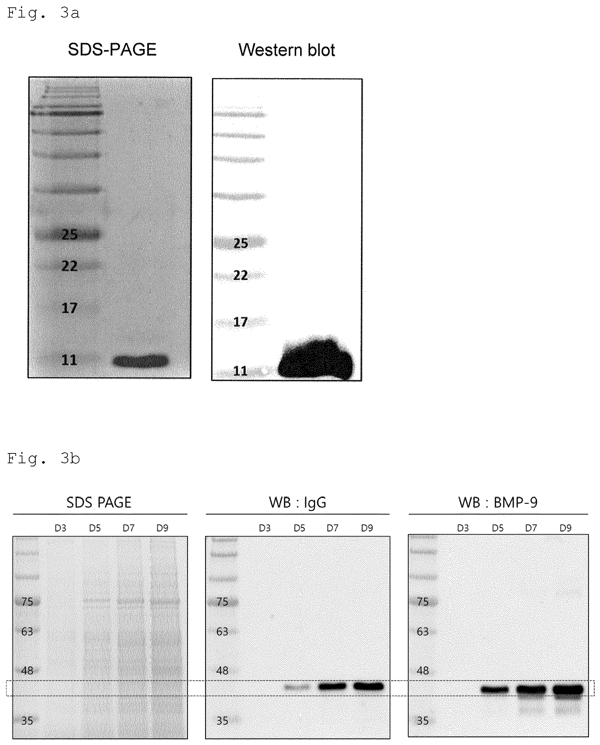

FIG. 3a shows results of SDS-PAGE and western blot of the expressed and purified BMP-9 variant, and FIG. 3b shows results of SDS-PAGE and western blot of the expressed and purified Fc-fused BMP-9.
FIG. 4 shows signaling activity (FIG. 4a) and ossification signaling activity (FIG. 4b) of the expressed and purified BMP-9 and variants on vascular endothelial cells.
FIG. 5 shows effects of BMP-9 variants on inhibiting fibrosis-related markers in myoblasts.

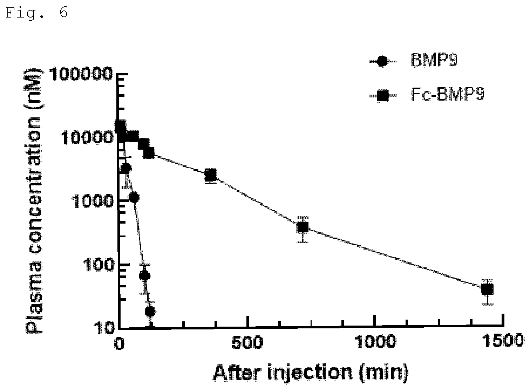

FIG. 6 is a graph showing results of measurement of blood concentrations of purified BMP-9 and Fc-fused BMP-9.

### Detailed Description and Preferred Embodiments of the Invention

Unless otherwise defined, all technical and scientific terms used herein have the same meanings as those typically understood by those skilled in the art to which the present invention belongs. In general, the nomenclature used herein and test methods described below are well known in the art and are typical.

In the present invention, a BMP-9 gene was cloned and various variants thereof were produced and then expressed and purified in mammalian cells (CHO cells and human fetal kidney cell lines), and thus a BMP-9 variant that is effective for tumors, inflammatory diseases, cardiovascular diseases, metabolic diseases, and autoimmune diseases was selected, and was fused with Fc, thus increasing the blood half-life thereof. Specifically, in the present invention, BMP-9 was designed to induce the optimal therapeutic effect by regulating the ability to bind to receptors through substitution of one or several amino acids in the amino acid sequences corresponding to the wrist epitope and the knuckle epitope thereamong. The present invention was intended to select a variant capable of suppressing ectopic ossification to one-tenth or less while maintaining endothelial-cell-specific signaling, and it was confirmed that the variant according to the present invention greatly reduced ectopic ossification in mouse myoblasts (C2C12) compared to the wild type through alkaline phosphatase activity and alizarin red staining.

Accordingly, an aspect of the present invention pertains to bone morphogenetic protein-9 (BMP-9) or a variant thereof represented by any one amino acid sequence selected from the group consisting of SEQ ID NOs: 1 to 29, and another aspect of the present invention pertains to a BMP-9 variant-Fc fusion protein in which an Fc fragment of an immunoglobulin is linked to the bone morphogenetic protein-9 (BMP-9) or the variant thereof.

The amino acid sequence of SEQ ID NO: 1 represents wild-type BMP-9, which comprises both the signal sequence in bold and the sequence of Pro-BMP-9 underlined as represented below. Among them, the sequence of FFPLADDVTPTKHAIVQTLVHLKF is a region that binds to ALK-1 and belongs to the wrist epitope when viewed from the overall BMP-9 structure, and the sequence of KVGKACCVPTKLSPISVLYK belongs to the knuckle epitope that binds to the BMP-2 receptor.

MAWVWTLLFLMAAAQSIQAKPLQSWGRGSAGGNAHSPLGVPGGGLPEHTFNLKMFLENVKVDFLRSLN
LSGVPSQDKTRVEPPQYMIDLYNRYTSDKSTTPASNIVRSFSMEDAISITATEDFPFQKHILLFNISIPRHEQIT
RAELRLYVSCQNHVDPSHDLKGSVVIYDVLDGTDAWDSATETKTFLVSQDIQDEGWETLEVSSAVKRWVRSDSTK
SKNKLEVTVESHRKGCDTLDISVPPGSRNLPFFVVFSNDHSSGTKETRLELREMISHEQESVLKKLSKDGSTEAG
ESSHEEDTDGHVAAGSTLARRKRSAGAGSHCQKTSLRVNFEDIGWDSWIIAPKEYEAYECKGGCFFPLADDVTPT
KHAIVQTLVHLKFPTKVGKACCVPTKLSPISVLYKDDMGVPTLKYHYEGMSVAECGCR

The variant sequence derived from the wild-type BMP-9 sequence may be represented by any one amino acid sequence selected from SEQ ID NOs: 2 to 29, and these variants are configured such that some amino acid sequences are substituted in the wild-type BMP-9 sequence. However, the variant according to the present invention is not limited to the specific amino acid sequence represented by the sequence ID number, and also, an amino acid sequence that may be considered equivalent to the corresponding amino acid sequence falls within the scope of the present invention, as will be apparent to those skilled in the art. For example, when the 335^{th} amino acid in SEQ ID NO: 2 is substituted with Ala, rather than Asp, compared to the wild type, so long as some of the sequences except for the core configuration of the variant are mutated so as not to affect the protein function and structure of BMP-9, the corresponding variant also falls within the scope of the present invention, as will be apparent to those skilled in the art. Accordingly, variants having at least 95%, at least 90%, at least 80%, or at least 70% homology with the remaining sequence other than the substitution of amino acids that are the core of the variant in any one amino acid sequence selected from SEQ ID NOs: 2 to 29 are understood to fall within the scope of the present invention.

Also, the variant of the present invention may be applied to other subtypes of bone morphogenetic protein, for example, BMP-7 or BMP-10.

In the present invention, in order to increase the blood half-life of wild-type or variant BMP-9, an Fc fragment of an immunoglobulin may be fused thereto. The Fc fragment of the immunoglobulin may be represented by the amino acid sequence of SEQ ID NO: 30, but is not limited thereto. In addition to the Fc fragment of the immunoglobulin, various peptides or proteins, such as albumin, may be fused thereto to increase the blood half-life of the protein.

In the present invention, the Fc fragment of the immunoglobulin may be linked to the N-terminus or C-terminus of the bone morphogenetic protein-9 (BMP-9) or the variant thereof.

In the present invention, the bone morphogenetic protein-9 (BMP-9) or the variant thereof and the Fc fragment of the immunoglobulin may be linked to each other via a linker, but the present invention is not limited thereto.

In the present invention, the linker may be represented by the amino acid sequence of SEQ ID NO: 31, but is not limited thereto.

In the present invention, the BMP-9 variant-Fc fusion protein may be represented by the amino acid sequence of SEQ ID NO: 32 or SEQ ID NO: 33, but is not limited thereto.

The BMP-9 or the variant thereof, or the fusion protein thereof according to the present invention may be used as a therapeutic, diagnostic, or research reagent, but is most preferably used for therapeutic purposes.

Specifically, the present invention provides a pharmaceutical composition for treating a tumor comprising the bone morphogenetic protein-9 (BMP-9) or the variant thereof, or the BMP-9 variant-Fc fusion protein, as an active ingredient.

In the present invention, the tumor may be at least one selected from the group consisting of breast cancer, lung cancer, colorectal cancer, colon cancer, liver cancer, pancreatic cancer, brain tumor, prostate cancer, skin cancer, osteosarcoma, and blood cancer, but is not limited thereto.

The present invention also provides a pharmaceutical composition for treating an inflammatory disease comprising the bone morphogenetic protein-9 (BMP-9) or the variant thereof, or the BMP-9 variant-Fc fusion protein, as an active ingredient.

In the present invention, the inflammatory disease may be at least one selected from the group consisting of steatohepatitis, hepatitis, and enteritis, but is not limited thereto.

The present invention also provides a pharmaceutical composition for treating a metabolic disease comprising the bone morphogenetic protein-9 (BMP-9) or the variant thereof, or the BMP-9 variant-Fc fusion protein, as an active ingredient.

In the present invention, the metabolic disease may be at least one selected from the group consisting of obesity, weight loss, diabetes, atherosclerosis, arteriosclerosis, cardiopulmonary disease, neurological disease, Alzheimer's disease, cognitive impairment, oxidative stress, skin disease, skin aging, damage caused by UV irradiation, hypertension, hypercholesterolemia (LDL, HDL, VLDL), hyperlipidemia (triglyceride), immunodeficiency, cancer, and metabolic syndrome, but is not limited thereto.

In the present invention, the cardiopulmonary disease may be at least one selected from the group consisting of myocardial infarction, hypertension, pulmonary arterial hypertension, myocardial fibrosis, and pulmonary fibrosis, but is not limited thereto.

The present invention also provides a pharmaceutical composition for treating an autoimmune disease comprising the bone morphogenetic protein-9 (BMP-9) or the variant thereof, or the BMP-9 variant-Fc fusion protein, as an active ingredient.

In the present invention, the autoimmune disease may be at least one selected from the group consisting of insulin-dependent diabetes, multiple sclerosis, autoimmune encephalomyelitis, rheumatoid arthritis, osteoarthritis, myasthenia gravis, thyroiditis, uveitis, Hashimoto's thyroiditis, thyrotoxicosis, pernicious anemia, autoimmune atrophic gastritis, autoimmune hemolytic anemia, idiopathic leukopenia, primary sclerosing cholangitis, alcoholic/nonalcoholic steatohepatitis, inflammatory bowel disease, Crohn's disease, ulcerative bowel disease, psoriasis, Sjogren's syndrome, scleroderma, Wegener's granulomatosis, polymyositis, dermatomyositis, discoid LE, and systemic lupus erythematosus, but is not limited thereto.

For the therapeutic purpose, the active BMP-9 variant according to the present invention or the derivative thereof may be administered alone, but is preferably administered in the form of a pharmaceutical composition (formulation), more preferably a sterile formulation.

In the present invention, the pharmaceutical composition may be formulated in any one dosage form selected from the group consisting of injectable preparations, oral preparations, liquids (e.g. for injection) such as aqueous solutions, suspensions, emulsions, etc., capsules, granules, tablets, and mucosal administration preparations, but the present invention is not limited thereto. These formulations may be prepared through a typical method used for formulation in the art or a method disclosed in Remington's Pharmaceutical Science (latest edition), Mack Publishing Company, Easton PA, and may be prepared into various formulations depending on individual diseases or components.

Also, the pharmaceutical composition of the present invention may further comprise at least one pharmaceutically acceptable carrier in addition to the therapeutic BMP-9 variant or the derivative thereof. The pharmaceutically acceptable carrier may be selected from among saline, sterile water, Ringer's solution, buffered saline, dextrose solution, maltodextrin solution, glycerol, ethanol, and mixtures of one or more thereof.

The pharmaceutical composition of the present invention may further comprise a pharmaceutically acceptable adjuvant, as necessary. The adjuvant may be at least one selected from the group consisting of excipients, diluents, dispersants, buffers, antimicrobial preservatives, bacteriostats, surfactants, binders, lubricants, antioxidants, thickeners, and viscosity modifiers, but is not limited thereto.

The pharmaceutical composition according to the present invention may be administered orally or parenterally (e.g. intravenously, subcutaneously, intramuscularly, intraperitoneally, or topically) depending on a desired method, and the dosage thereof may be variously changed and applied depending on the patient's weight, age, gender, health status, and diet, administration time, administration method, excretion rate, and severity of disease according to the opinion of an expert.

In an embodiment of the present invention, a single dosage of the protein may be 1 µg/kg to 100 mg/kg, preferably 5 µg/kg to 50 mg/kg, and the protein may be administered once a day or 1-3 times a week, but the dosage and administration interval are not limited thereto.

Still another aspect of the present invention pertains to a method of preventing or treating a tumor, an inflammatory disease, a metabolic disease, or an autoimmune disease, comprising administering the BMP-9 variant or the BMP-9 variant-Fc fusion protein.

Yet another aspect of the present invention pertains to the use of the BMP-9 variant or the BMP-9 variant-Fc fusion protein for the prevention or treatment of a tumor, an inflammatory disease, a metabolic disease, or an autoimmune disease.

Still yet another aspect of the present invention relates to the use of the BMP-9 variant or the BMP-9 variant-Fc fusion protein for the manufacture of a medicament for the treatment of a tumor, an inflammatory disease, a metabolic disease, or an autoimmune disease.

### Numbered embodiments of the invention

Numbered embodiment 1: A bone morphogenetic protein-9 (BMP-9) variant represented by any one amino acid sequence selected from the group consisting of SEQ ID NOs: 2 to 29.

Numbered embodiment 2: A BMP-9 variant-Fc fusion protein in which an Fc fragment of an immunoglobulin is linked to the BMP-9 variant according to Numbered embodiment 1.

Numbered embodiment 3: The BMP-9 variant-Fc fusion protein according to Numbered embodiment 2, wherein the Fc fragment of the immunoglobulin is represented by an amino acid sequence of SEQ ID NO: 30.

Numbered embodiment 4: The BMP-9 variant-Fc fusion protein according to Numbered embodiment 2, wherein the BMP-9 variant and the Fc fragment of the immunoglobulin are linked to each other via a linker.

Numbered embodiment 5: The BMP-9 variant-Fc fusion protein according to Numbered embodiment 4, wherein the linker is represented by an amino acid sequence of SEQ ID NO: 31.

Numbered embodiment 6: A pharmaceutical composition for treating a tumor comprising the BMP-9 variant according to claim 1 or the BMP-9 variant-Fc fusion protein according to any one of Numbered embodiments 2 to 5 as an active ingredient.

Numbered embodiment 7: The pharmaceutical composition according to Numbered embodiment 6, wherein the tumor is at least one selected from the group consisting of breast cancer, lung cancer, colorectal cancer, colon cancer, liver cancer, pancreatic cancer, brain tumor, prostate cancer, skin cancer, osteosarcoma, and blood cancer.

Numbered embodiment 8: A pharmaceutical composition for treating an inflammatory disease comprising the BMP-9 variant according to Numbered embodiment 1 or the BMP-9 variant-Fc fusion protein according to any one of Numbered embodiments 2 to 5 as an active ingredient.

Numbered embodiment 9: The pharmaceutical composition according to Numbered embodiment 8, wherein the inflammatory disease is at least one selected from the group consisting of steatohepatitis, hepatitis, and enteritis.

Numbered embodiment 10: A pharmaceutical composition for treating a metabolic disease comprising the BMP-9 variant according to Numbered embodiment 1 or the BMP-9 variant-Fc fusion protein according to any one of Numbered embodiments 2 to 5 as an active ingredient.

Numbered embodiment 11: The pharmaceutical composition according to Numbered embodiment 10, wherein the metabolic disease is at least one selected from the group consisting of obesity, weight loss, diabetes, atherosclerosis, arteriosclerosis, cardiopulmonary disease, neurological disease, Alzheimer's disease, cognitive impairment, oxidative stress, skin disease, skin aging, damage caused by UV irradiation, hypertension, hypercholesterolemia (LDL, HDL, VLDL), hyperlipidemia (triglyceride), immunodeficiency, cancer, and metabolic syndrome.

Numbered embodiment 12: The pharmaceutical composition according to Numbered embodiment 11, wherein the cardiopulmonary disease is at least one selected from the group consisting of myocardial infarction, hypertension, pulmonary arterial hypertension, myocardial fibrosis, and pulmonary fibrosis.

Numbered embodiment 13: A pharmaceutical composition for treating an autoimmune disease comprising the BMP-9 variant according to Numbered embodiment 1 or the BMP-9 variant-Fc fusion protein according to any one of Numbered embodiments 2 to 5 as an active ingredient.

Numbered embodiment 14: The pharmaceutical composition according to Numbered embodiment 13, wherein the autoimmune disease is at least one selected from the group consisting of insulin-dependent diabetes, multiple sclerosis, autoimmune encephalomyelitis, rheumatoid arthritis, osteoarthritis, myasthenia gravis, thyroiditis, uveitis, Hashimoto's thyroiditis, thyrotoxicosis, pernicious anemia, autoimmune atrophic gastritis, autoimmune hemolytic anemia, idiopathic leukopenia, primary sclerosing cholangitis, alcoholic/nonalcoholic steatohepatitis, inflammatory bowel disease, Crohn's disease, ulcerative bowel disease, psoriasis, Sjogren's syndrome, scleroderma, Wegener's granulomatosis, polymyositis, dermatomyositis, discoid LE, and systemic lupus erythematosus.

### Examples

A better understanding of the present invention may be obtained through the following examples. These examples are merely set forth to illustrate the present invention, and are not to be construed as limiting the scope of the present invention, as will be apparent to those skilled in the art.

### Example 1. Construction of recombinant human proBMP-9 and proBMP-10

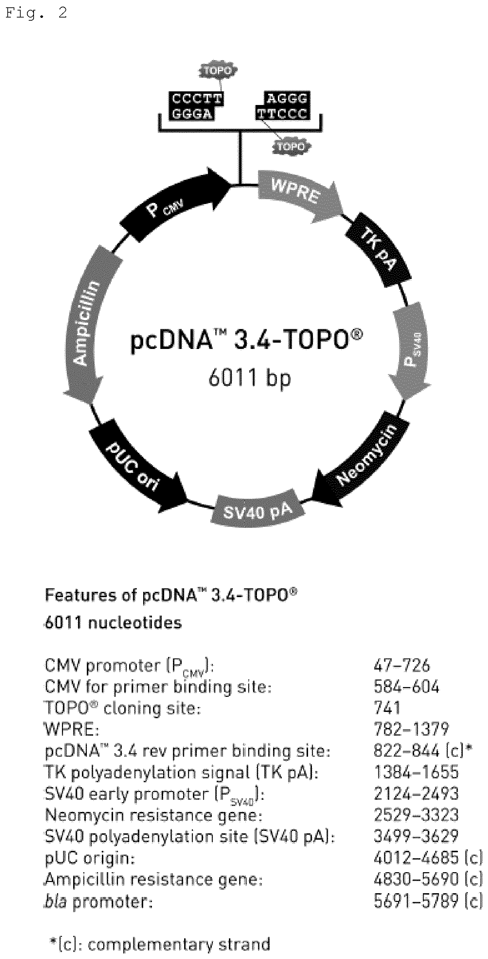

The entire range of cDNA including the open reading frame of human pre-proBMP9 was inserted and cloned into a pcDNA3.4 vector (FIG. 2), which was confirmed by DNA sequencing. The variant of proBMP9 was obtained using the QuickChange Site-directed mutagenesis kit, which was also confirmed by DNA sequencing.

BMP-9 wild-type and variant sequences used in the present invention are as follows.

SEQ ID NO: 1. wild-type latent BMP-9 (NIBEC-J)

MAWVWTLLFLMAAAQSIQAKPLQSWGRGSAGGNAHSPLGVPGGGLPEHTFNLKMFLENVKVDFLRSLN
LSGVPSQDKTRVEPPQYMIDLYNRYTSDKSTTPASNIVRSFSMEDAISITATEDFPFQKHILLFNISIPRHEQIT
RAELRLYVSCQNHVDPSHDLKGSVVIYDVLDGTDAWDSATETKTFLVSQDIQDEGWETLEVSSAVKRWVRSDSTK
SKNKLEVTVESHRKGCDTLDISVPPGSRNLPFFVVFSNDHSSGTKETRLELREMISHEQESVLKKLSKDGSTEAG
ESSHEEDTDGHVAAGSTLARRKRSAGAGSHCQKTSLRVNFEDIGWDSWIIAPKEYEAYECKGGCFFPLADDVTPT
KHAIVQTLVHLKFPTKVGKACCVPTKLSPISVLYKDDMGVPTLKYHYEGMSVAECGCR

In the wild-type sequence, the signal peptide sequence is represented in bold, the Pro-BMP sequence is underlined, and the BMP-9 sequence is unmarked.

The plasmid containing pre-proBMP9 was transfected with polyethyleneimine into a CHO-S cell line, and an enhancer was then added thereto, followed by culture for 8 days. The expressed proBMP9 and proBMP10 were measured using an antiBMP9 antibody and an antiBMP10 antibody through a western blot method.

The expressed protein was isolated through a Q-Sepharose column that had already reached equilibrium using 1-5 L of conditioned medium as a buffer. The target protein attached to the column was subjected to fractionation by a sodium chloride gradient, and the fractionated sample was concentrated and then subjected to gel chromatography to obtain a target molecular weight. The protein thus obtained was confirmed to have a purity of 95% or more.

### Example 2. Construction of recombinant human proBMP-9 variants

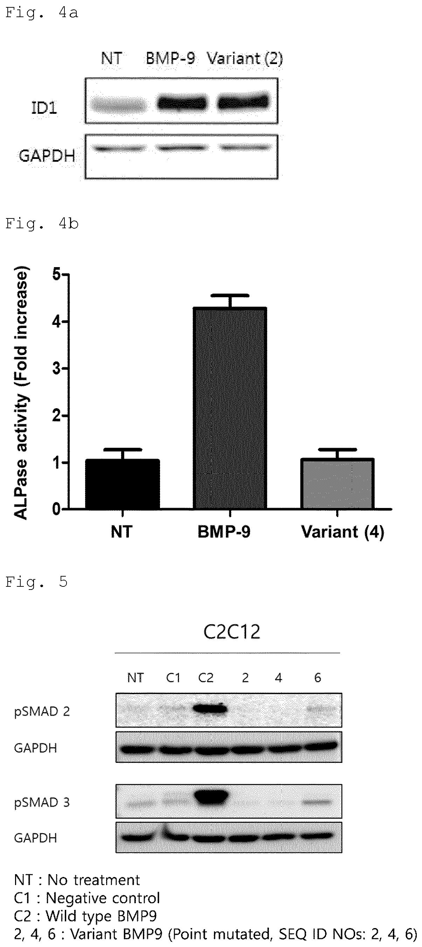

BMP-9 or BMP-10 selectively binds to the ALK1 receptor in vascular endothelial cells, and thus, is promising as a therapeutic agent for cardiovascular diseases. However, they still have the potential to stimulate ossification by stimulating mesenchymal cells or myoblasts. When developing a therapeutic agent, it is necessary to incapacitate such potential. To date, it is not known which receptors are involved in the osteogenic differentiation capacity of BMP-9 or BMP-10. The researchers of the present invention have identified that the wrist epitope and the knuckle epitope in the structure of BMP-9 act as ligands for mediating major signaling, confirming that, by regulating these sequences, osteogenic differentiation capacity can be suppressed while maintaining endothelial cell signaling (SEQ ID NOs: 1-29, FIG. 4). These variants were determined to have similar physiological activities *in vivo,* and were expected to provide the advantage of enhancing the therapeutic effect while minimizing concerns about side effects.

### Example 3. Signaling by BMP-9 variants and derivatives in endothelial cells

The amount of each proBMP9 variant was measured through ELISA and cells were treated therewith. In a serum-free state, the variant derived above was added to HUVEC cells in the indicated concentration range. After 8 hours of treatment, the cells were harvested, mRNA was extracted therefrom, and the expression of ID1 and BMPR-II was measured through quantitative PCR. In addition, for the expression of pSmad1/5/8, the cells were treated with the BMP-9 variant in a serum-free state, and after 1 hour, the results thereof were observed. The expression level of the protein obtained after treatment of the cells in a lysis buffer was measured through immunoblotting against the anti-pSmad1/5/8 antibody. Based on the results of observation (FIG. 4), it was confirmed that the variants significantly increased the expression of ID1 and BMPR-II, and the increased amount did not show a great difference compared to the wild-type BMP-9. As seen in previous reports, BMP-9 or BMP-10 was confirmed to act as ligands of ALK1 receptors in vascular endothelial cells. According to literature, BMP-9 is known to inhibit endothelial cell migration, proliferation, and angiogenesis by endothelial cells in the circulatory system.

### Example 4. Osteogenic differentiation signaling by BMP-9 variants in C2C12 cell culture

C2C12, a mouse myoblast, was selected to observe osteogenic differentiation capacity because it has differentiation ability similar to that of mesenchymal stem cells. C2C12 cells were cultured for 16 hours in DMEM containing 0.25% FBS and then treated with BMP-9 variants. After further culture for 72 hours, the cells were lysed with 1% Triton X-100/PBS, and the resulting protein was measured for ALP enzyme activity. ALP enzyme activity was determined by measuring absorbance at 405 nm of a watersoluble material produced by reacting with a 4-nitrophenyl phosphate disodium salt, which is a substrate of the enzyme, and BMP-9 was purchased as a standard material and compared therewith. Based on the results of observation, it was confirmed that the variants had greatly lowered osteogenic differentiation capacity compared to the standard material (FIG. 4). The variants thus formed were judged to provide a therapeutic effect and also to have no side effects of ectopic ossification, by incapacitating ossification while maintaining the vascular endothelial cell signaling in Example 3.

### Example 5. Effect of prolonging half-life of BMP-9-Fc derivative in vivo

Each of BMP-9 and Fc-fused BMP-9 derivative was injected at a dose of 1 mg/kg through the tail vein of mice, blood was collected at regular time intervals, and the amount of BMP-9 in the blood was measured using a BMP-9 quantitative kit. Based on the results of measurement (FIG. 5), the Fc-bound BMP-9 derivative (SEQ ID NOs: 32-33) showed a greatly increased blood concentration curve.

Although specific embodiments of the present invention have been disclosed in detail above, it will be obvious to those skilled in the art that the description is merely of preferable exemplary embodiments and is not to be construed as limiting the scope of the present invention. Therefore, the substantial scope of the present invention will be defined by the appended claims and equivalents thereto.

### Industrial Applicability

According to the present invention, a variant was designed so as to maximize the therapeutic effect of BMP-9 and greatly reduce side effects thereof, and was expressed and purified with high efficiency in mammalian cells. Moreover, in order to extend the blood half-life of BMP-9, a fusion protein fused with the Fc region of immunoglobulin was additionally constructed. Such a variant and fusion protein can exhibit superior therapeutic effects on various diseases such as tumors, fibrosis, cardiopulmonary vascular disease, obesity, and fatty liver *in vitro* and through animal experimentation, and thus can be expected to be useful as novel therapeutic agents for the treatment of these diseases.

### Sequence List Free Text

An electronic file is attached.

## Claims

1. A bone morphogenetic protein-9 (BMP-9) variant represented by an amino acid sequence of SEQ ID NOs: 4.

2. A BMP-9 variant-Fc fusion protein in which an Fc fragment of an immunoglobulin is linked to the BMP-9 variant according to claim 1.

3. The BMP-9 variant-Fc fusion protein according to claim 2, wherein the Fc fragment of the immunoglobulin is represented by an amino acid sequence of SEQ ID NO: 30.

4. The BMP-9 variant-Fc fusion protein according to claim 2, wherein the BMP-9 variant and the Fc fragment of the immunoglobulin are linked to each other via a linker.

5. The BMP-9 variant-Fc fusion protein according to claim 4, wherein the linker is represented by an amino acid sequence of SEQ ID NO: 31.

6. A composition for use in treatment of a tumor comprising the BMP-9 variant according to claim 1 or the BMP-9 variant-Fc fusion protein according to any one of claims 2 to 5 as an active ingredient.

7. The composition for use according to claim 6, wherein the tumor is at least one selected from the group consisting of breast cancer, lung cancer, colorectal cancer, colon cancer, liver cancer, pancreatic cancer, brain tumor, prostate cancer, skin cancer, osteosarcoma, and blood cancer.

8. A composition for use in treatemnt of an inflammatory disease comprising the BMP-9 variant according to claim 1 or the BMP-9 variant-Fc fusion protein according to any one of claims 2 to 5 as an active ingredient.

9. The composition for use according to claim 8, wherein the inflammatory disease is at least one selected from the group consisting of steatohepatitis, hepatitis, and enteritis.

10. The composition for use according to claim 9, wherein steatohepatitis is alcoholic steatohepatitis or nonalcoholic steatohepatitis.

11. A composition for use in treatment of a metabolic disease comprising the BMP-9 variant according to claim 1 or the BMP-9 variant-Fc fusion protein according to any one of claims 2 to 5 as an active ingredient.

12. The pharmaceutical composition according to claim 11, wherein the metabolic disease is at least one selected from the group consisting of obesity, diabetes, atherosclerosis, arteriosclerosis, neurological disease, Alzheimer's disease, hypertension, hypercholesterolemia, hyperlipidemia, immunodeficiency, and metabolic syndrome.

13. A composition for use in treatment of cardiopulmonary disease comprising the BMP-9 variant according to claim 1 or the BMP-9 variant-Fc fusion protein according to any one of claims 2 to 5 as an active ingredient, wherein the cardiopulmonary disease is at least one selected from the group consisting of myocardial infarction, hypertension, pulmonary arterial hypertension, myocardial fibrosis, and pulmonary fibrosis.

14. A composition for use in treatment of an autoimmune disease comprising the BMP-9 variant according to claim 1 or the BMP-9 variant-Fc fusion protein according to any one of claims 2 to 5 as an active ingredient.

15. The composition for use according to claim 14, wherein the autoimmune disease is at least one selected from the group consisting of insulin-dependent diabetes, multiple sclerosis, autoimmune encephalomyelitis, rheumatoid arthritis, osteoarthritis, myasthenia gravis, thyroiditis, uveitis, Hashimoto's thyroiditis, thyrotoxicosis, pernicious anemia, autoimmune atrophic gastritis, autoimmune hemolytic anemia, idiopathic leukopenia, primary sclerosing cholangitis, inflammatory bowel disease, Crohn's disease, ulcerative bowel disease, psoriasis, Sjogren's syndrome, scleroderma, Wegener's granulomatosis, polymyositis, dermatomyositis, discoid LE, and systemic lupus erythematosus.
